# DEMANDE DE BREVET EUROPEEN

(11) **EP 0 544 045 A1**
(43) Date de publication de la demande: **02.06.1993**
(21) Numéro de dépôt: 91403223.0
(22) Date de dépôt: 28.11.1991
(51) Int. Cl.: A61F 13/14

(54) **Coussinet d'allaitement**

(71) Demandeur: AQUILON PRODUCTION Sarl, F-59118 Wambrechies (FR)
(72) Inventeur: Vandewalle, Monique, F-59350 Saint Andre (FR)
(74) Mandataire: Prignot, Jean

(57) **Abrégé**

L'invention a pour objet un coussinet d'allaitement.

Le coussinet d'allaitement en un matériau absorbant, constitué d'un élément plan (2) présentant au moins une découpe (3) issue de sa périphérie et s'étendant substantiellement vers sa partie centrale pour permettre lors de l'utilisation, par modification de la position relative des bords de la découpe (3) la constitution d'un coussinet de forme générale cônique adapté à la forme du sein, est, suivant l'invention, dépourvu de tout élément de fixation de la position relative des bords de la découpe (3) susceptible d'empêcher une modification de cette position relative, et donc de la forme du coussinet, sous l'effet du gonflement du sein à la montée du lait.

## Description

La présente invention a pour objet un coussinet d'allaitement de grand confort et de grande efficacité, et en outre de fabrication aisée.

Les coussinets d'allaitement, qui sont des éléments absorbants destinés à recueillir les pertes de lait qui se produisent lors de la montée du lait chez les femmes qui allaitent, sont à ce jour généralement réalisés sous forme d'éléments circulaires découpés par estampage dans un produit absorbant multicouches en forme de feuille. Ces éléments sont formés en dôme lors de l'estampage, pour s'adapter à la forme du sein, et leur périphérie est soudée ou compressée pour assurer la cohésion complète du coussinet sur ses bords.

Le rôle des coussinets d'allaitement est double, et consiste d'une part à éviter que les vêtements soient tâchés par les pertes de lait, et d'autre part à maintenir autant que possible le bout du sein sec afin d'éviter irritations et crevasses.

Les coussinets d'allaitement connus remplissent imparfaitement ce rôle. Un de leurs principaux inconvénients est leur défaut d'adaptation au sein. La raison en est que la forme de dôme qui leur est conférée lors de la fabrication est une forme moyenne, qui ne correspond généralement que sensiblement à la forme du sein auquel le coussinet doit s'adapter.

Des coussinets de ce type, mais toutefois réalisés à partir de disques plans découpés et assemblés en cône à la fabrication, sont déjà connus par exemple par les brevets GB-A-2064961 et GB-A-904580.

Il a par ailleurs déjà été proposé au brevet US-A-3438362 de constituer des coussinets d'allaitement en forme de disques plan comportant une découpe en forme de secteur circulaire, disques destinés à être formés en coussinet d'allaitement conique sur le sein.

La présente invention a pour objet des coussinets d'allaitement de ce type.

Ce document antérieur enseigne de former sur une face du disque une bande adhésive destinée d'une part à permettre le maintien en place et la fixation du coussinet d'allaitement dans le soutien-gorge, et d'autre part la fixation l'une à l'autre des régions des bords de la découpe par recouvrement de la partie non-adhésive sur la partie adhésive. De la sorte, la forme cônique du coussinet constitué est maintenue sans possibilité de modification, tout au long de son utilisation.

De tels coussinets, parfaitement adaptés à la forme du sein lors du placement, ne sont cependant pas encore en mesure de maintenir leur adaptation au sein du fait que, lors de la montée du lait, le sein gonfle et donc sa forme se modifie. Les conséquences de cette inadaptation à la forme du sein sont la difficulté de maintenir correctement en place le coussinet, et donc le risque que les pertes de lait ne soient pas correctement absorbées. Dans le brevet US-A-3438362, ce maintien en place du coussinet est assuré par la fixation au soutien-gorge.

La présente invention a pour objet de fournir un coussinet d'allaitement qui demeure à tout moment adapté à la forme du sein, et dont le maintien en place est donc assuré sans qu'une quelconque fixation à un vêtement soit nécessaire.

Dans ce but, le coussinet de l'invention réalisé sous forme d'un élément plan, produit de fabrication et d'emballage aisés, susceptible d'être amené à la forme du sein sur le sein lui-même, et ainsi de s'adapter à ce dernier, a été conçu pour suivre les modifications de forme du sein lors de son gonflement durant les montées de lait.

Ce but est atteint en prévoyant un coussinet d'allaitement en un matériau absorbant qui est constitué d'un élément plan présentant au moins une découpe issue de sa périphérie et s'étendant substantiellement vers sa partie centrale pour permettre, lors de l'utilisation, par modification de la position relative des bords de la découpe, la constitution d'un coussinet de forme générale cônique adapté à la forme du sein, ce coussinet étant dépourvu de tout élément de fixation de la position relative des bords de la découpe susceptible d'empêcher une modification de cette position relative, et donc de la forme du coussinet, sous l'effet du gonflement du sein à la montée du lait.

Il a en effet été constaté que l'étanchéité du coussinet est préservée par simple recouvrement des bords de la découpe, sans que ceux-ci doivent être fixés l'un à l'autre.

Suivant une caractéristique de l'invention, la découpe est en forme de fente.

Suivant une autre caractéristique de l'invention, la découpe a une forme triangulaire à la manière d'un secteur circulaire.

Suivant une caractéristique supplémentaire, la découpe issue de la périphérie est une fente s'étendant suivant une direction générale diamétrale, sur une distance comprise entre 30 et 60% du diamètre du disque.

Suivant une caractéristique supplémentaire, dans la région centrale du disque, la découpe issue de la périphérie affecte sur environ 180° une forme circulaire, concentrique au disque.

Suivant une autre caractéristique de l'invention, l'élément plan en matière absorbante est solidaire, sur sa face externe, d'une feuille imperméable.

Suivant une autre caractéristique de l'invention, la découpe de l'élément s'étend à la feuille imperméable.

Suivant encore une autre caractéristique de l'invention, la découpe en forme de secteur de l'élément ne s'étend pas à la feuille imperméable.

Suivant une autre caractéristique de l'invention, du fil élastique posé à l'état tendu est rendu solidaire de l'élément plan de part et d'autre des bords de la découpe.

Enfin, suivant encore un caractéristique de l'invention, les bords du coussinet présentent des points de repère visuels sous forme de motifs découpés ou de motifs imprimés pour faciliter la mise en forme immédiate du coussinet, de manière adaptée à la taille ou à la forme du sein.

L'invention sera mieux comprise en se reportant à la description, en même temps qu'au dessin annexé qui représente, uniquement à titre d'exemple, divers modes de réalisation de l'invention et dans lequel:
- la fig 1 est une vue en plan d'un mode de réalisation de l'invention,
- les fig 2 et 3 représentent, en perspective, le coussinet d'allaitement de la fig 1 tel qu'il est susceptible d'être formé sur le sein
- les fig 4 à 12 représentent en plan différents autres modes de réalisation de l'invention.

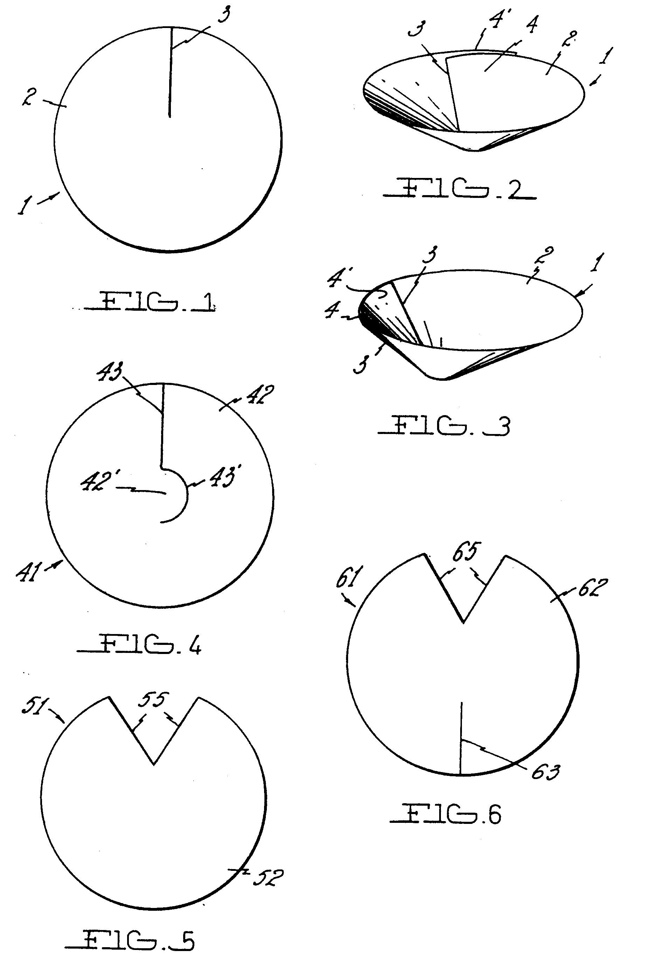

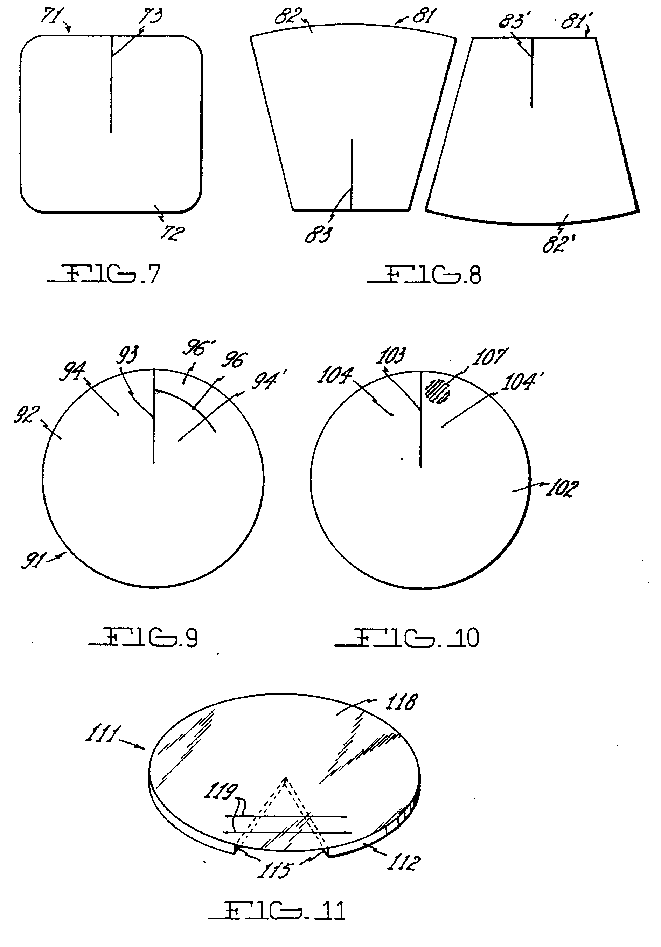

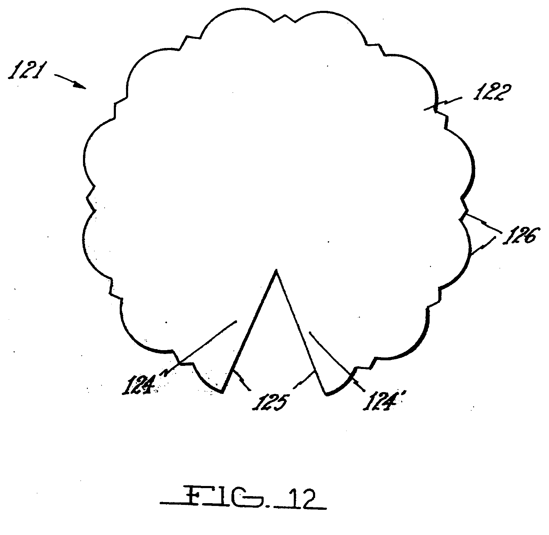

En se reportant à la fig 1, un coussinet 1 est constitué d'un élément plan 2 en forme de disque, pourvu d'une découpe sous la forme d'une fente 3 radiale, et est dépourvu de tout élément permettant de fixer la position relative des bords de la fente. Lors de l'utilisation, comme illustré aux figures 2 et 3, on amène par superposition des zones 4, 4' de l'élément plan, adjacentes à la fente 3, l'élément plan 2 à une forme générale conique, dont l'angle au sommet dépend du recouvrement réalisé dans les zones 4, 4'. De la sorte, en formant l'élément directement sur le sein, on conçoit qu'il s'adapte à la forme de ce dernier. En utilisation, la forme générale conique du coussinet est maintenue par coopération de ce dernier avec la poche du soutien-gorge qui le recouvre.

Etant donné que le cousinet est dépourvu de tout élément de fixation de la position relative des bords de la fente, lors des montées de lait, lorsque le sein gonfle le coussinet est susceptible d'adapter sa forme à ce gonflement par glissement des zones 4, 4' l'une par rapport à l'autre, restant ainsi adapté à la forme du sein.

En se reportant à la fig 3, le fait de limiter la fente 3 à une partie seulement du rayon du disque 2 assure au coussinet 1, lors de sa formation en un élément conique, une dissymétrie qui peut favoriser l'adaptation du coussinet à la forme du sein.

Suivant le mode de réalisation illustré à la fig 4, la découpe 43 en forme de fente du disque 42 se prolonge en une découpe circulaire 43' assurant, lors de l'adaptation du coussinet au sein, la formation d'une partie plane 42' de forme correspondant à celle du bout du sein.

Le coussinet 51 suivant le mode de réalisation de la fig 5 est constitué d'un élément plan 52, dans lequel est formée une découpe triangulaire 55, à la manière d'un secteur circulaire.

Le coussinet 61, suivant le mode de réalisation de la fig 6, comporte à la fois une découpe triangulaire 65 et une découpe en forme de fente 63, disposées de part et d'autre du centre du disque 62.

L'élément plan constituant le coussinet peut bien entendu présenter d'autres formes que la forme circulaire.

C'est ainsi qu'est illustré à la fig 7 un coussinet 71 constitué d'un élément plan de forme générale polygonale 72 pourvu d'une découpe en forme de fente 73.

De même la fig 8 représente deux coussinets 81, 81', juxtaposés de la manière dont ils se présentent à l'étape de fabrication. Suivant ce mode de réalisation, la forme en secteur circulaire tronqué des éléments 82, 82' est particulièrement avantageuses du point de vue de l'économie de matière, lors de la découpe des dits éléments 82, 82' à partir d'un matériau en feuille. Les éléments 82, 82' sont pourvus de découpes 83, 83' en forme de fentes.

On peut également prévoir des dispositifs permettant un meilleur maintien du coussinet à l'état formé. Des dispositifs de ce genre ont été illustrés à titre d'exemple aux fig 9 et 10.

Suivant le mode de réalisation de la fig 9, un coussinet 91 formé d'un élément plan 92 pourvu d'une découpe en forme de fente 93, présente dans sa zone 94' une seconde découpe 96 aboutissant à la fente 92 et constituant une languette 96'. Lors de la formation du coussinet 91, par superposition des zones 94 et 94', la languette 96' et le restant de la zone 94' sont disposés de part et d'autre de la zone 94, assurant ainsi une meilleure retenue de la zone 94 sur la zone 94'. Cette retenue est toutefois insuffisante pour empêcher une modification de la position relative des bords de la découpe 93 sous l'effet du gonflement du sein à la montée du lait.

Suivant le mode de réalisation de la fig 10, il est prévu sur la zone 104' adjacente à la fente 103 de l'élément plan 102, un point de colle ou adhésif illustré en 107. Lors de la mise en forme du coussinet 101 par superposition des zones 104 et 104', le point de colle 107 assure une liaison entre les dites zones. Les dimensions du point de colle ou de l'adhésif ainsi que la force du collage sont choisis pour que cette liaison cède sous l'effet du gonflement du sein à la montée du lait.

Le coussinet de l'invention sera de préférence réalisé à partir d'une feuille d'un complexe souple absorbant, qui contient un matériau absorbant du type ouate, fluff ou papier, et qui est susceptible de contenir une matière super-absorbante actuellement connue dans le public sous les termes "mini-capteurs".

Généralement ce matériau sera complété, sur une de ses faces, par une feuille imperméable.

Les différents modes de réalisation de l'invention illustrés aux fig 1 à 10 peuvent être réalisés en une seule opération, par découpe rotative, à partir d'un matériau en feuille, comportant éventuellement une face imperméable, auquel cas la découpe concerne également cette face imperméable.

On peut toutefois également concevoir que la feuille imperméable soit rendue solidaire du matériau après réalisation de la découpe, et seulement sur une partie de la surface du coussinet, la feuille imperméable n'étant pas rendue solidaire du coussinet dans les zones adjacentes à la fente ou à la découpe.

La fig 11 illustre un mode de réalisation d'un tel coussinet 111, constitué d'un élément plan 112 pourvu d'une découpe triangulaire 115. Après découpe, cet élément a été rendu solidaire d'une feuille imperméable 118. Des fils élastiques 119, posés à l'état tendu, ont été rendus solidaires de la feuille imperméable, et donc de l'élément plan 112, de part et d'autre de la découpe 115. Lorsque la tension maintenant les fils élastiques à l'état tendu est relâchée, le coussinet 111 adopte la forme qu'il aura en utilisation, par rapprochement/superposition des bords de la découpe 115. Ces fils élastiques n'empêchent pas la modification de la forme du coussinet par glissement l'un sur l'autre des bords de la découpe, lors du gonflement du sein à la montée du lait, mais au contraire assurent un parfait maintien du coussinet au contact du sein.

Enfin, la fig 12 illustre un coussinet 121, constitué d'un élément plan 122 de forme généralement circulaire, dont les bords présentent un décor. Dans l'exemple illustré ce décor est un motif découpé 126, mais il pourrait également être constitué d'un motif imprimé ou d'un motif de dentelle. De tels décors, outre qu'ils agrémentent, d'un point de vue esthétique, l'aspect du coussinet, ont pour fonction de servir de points de repère, lors de la mise en forme du produit, pour déterminer la dimension, la forme et le volume du cône. Ils facilitent ainsi visuellement et de manière immédiate la mise en forme du coussinet, adaptée à la taille du sein, lors de la superposition des zones 124, 124' adjacentes à la découpe 125.

Bien que dans les différents modes de réalisation illustré, la découpe soit essentiellement radiale, c'est-à-dire qu'elle s'étend généralement selon une direction allant sensiblement du centre de l'élément à sa périphérie, on peut également concevoir qu'elle soit tangente à une zone centrale de dimensions plus ou moins importante. On considère qu'une mise en forme satisfaisante peut être obtenue pour des découpes dont la projection sur la droite, issue de l'origine de la découpe à la périphérie de l'élément et passant par le centre de l'élément, s'étend sur une distance correspondant à 10 à 90% de la dimension de l'élément suivant cette droite. La découpe peut être en une partie, comme illustré dans la plupart des modes de réalisation ci-dessus, ou en deux parties comme illustré à la fig 6.

Les modes de réalisation préférés de l'invention sont toutefois des coussinets formés d'une élément en forme de disque, dans lequel la découpe est une découpe en forme de fente suivant une direction diamétrale, issue de la périphérie de disque et s'étendant sur une distance correspondant à 30 à 60% du diamètre du disque.

## Revendications

1. Coussinet d'allaitement en un matériau absorbant, constitué d'un élément plan (2;42;52;62;72;82,82';92;102; 112;122) présentant au moins une découpe (3;43,43';55; 63,65;73;83,83';93;103;115;125) issue de sa périphérie et s'étendant substantiellement vers sa partie centrale pour permettre lors de l'utilisation, par modification de la position relative des bords de la découpe (3;43,43';55; 63,65;73;83,83';93;103;115;125), la constitution d'un coussinet de forme générale cônique adapté à la forme du sein, caractérisé en ce que le coussinet est dépourvu de tout élément de fixation de la position relative des bords de la découpe (3;43,43';55;63,65;73;83,83';93;103;115;125) susceptible d'empêcher une modification de cette position relative, et donc de la forme du coussinet, sous l'effet du gonflement du sein à la montée du lait.

2. Coussinet d'allaitement suivant la revendication 1, caractérisé en ce que la découpe (3;43,43';63;73;83,83'; 93;103) est en forme de fente.

3. Coussinet d'allaitement suivant la revendication 1, caractérisé en ce que la découpe (55;65;115;125) est de forme triangulaire, à la manière d'un secteur circulaire.

4. Coussinet d'allaitement suivant la revendication 2, caractérisé en ce que la découpe issue de la périphérie est une fente (3;43;63;93;103) s'étendant suivant une direction générale diamétrale sur une distance comprise entre 30 et 60% du diamètre du disque.

5. Coussinet d'allaitement suivant la revendication 4, caractérisé en ce que, dans la région centrale du disque, la découpe (43,) issue de la périphérie, affecte sur environ 180° une forme circulaire (43'), concentrique au disque.

6. Coussinet d'allaitement suivant l'une quelconque des revendications précédentes, caractérisé en ce que l'élément plan (112) en matière absorbante est solidaire, sur sa face externe, d'une feuille imperméable (118).

7. Coussinet d'allaitement suivant la revendication 6, caractérisé en ce que la découpe de l'élément s'étend à la feuille imperméable.

8. Coussinet d'allaitement suivant la revendication 6, caractérisé en ce que la découpe (115) en forme de secteur de l'élément (112) ne s'étend pas à la feuille imperméable (118).

9. Coussinet d'allaitement suivant l'une quelconque des revendications précédentes, caractérisé en ce que du fil élastique, posé à l'état tendu, est rendu solidaire de l'élément plan de part et d'autre de la découpe.

10. Coussinet d'allaitement suivant l'une quelconque des revendications 1 à 9, caractérisé en ce que les bords de l'élément (122) plan présentent des points de repère visuels sous forme de motifs découpés (126) ou de motifs imprimés pour faciliter la mise en forme immédiate du coussinet, de manière adaptée à la taille ou à la forme du sein.
